# EUROPEAN PATENT APPLICATION

(11) **EP 2 777 719 A2**
(43) Date of publication of application: **17.09.2014**
(21) Application number: 14157335.2
(22) Date of filing: 28.02.2014
(51) Int. Cl.: A61L 15/44, A61L 15/46, A61L 15/60

(54) **Preparation Patch and Safety Syringe System**

(30) Priority: 14.03.2013 US 201361782506 P
(71) Applicant: Covidien LP, Mansfield, MA 02048 (US)
(72) Inventor: Finke, Mel, DeLand, FL 32720 (US); Heagle, David, Providence, RI 02904 (US); Boulanger, Jason, Trenton, IL 62293 (US); Silva, Benjamin, San Francisco, CA 94109 (US); Copp-Howland, Warren, Chicopee, MA 01013 (US); Garstka, Erick, Westfield, MA 01085 (US); Tremblay, Kathleen, Westfield, MA 01085 (US); Sellechio, Michael, Cranston, RI 02910 (US)
(74) Representative: Olsson, Carl H.S.

(57) **Abstract**

A safety syringe system including a safety syringe and an injection site preparation patch adapted for use with the safety syringe. The safety syringe includes a syringe body and a safety shield slidably disposed about the syringe body. The injection site preparation patch has a tissue-facing side and a syringe-facing side, and may include a substrate, a syringe adhesive layer adapted to adhere to a distal flange of the safety shield, a hydrogel layer adapted to adhere to tissue, and removable release liners disposed on either side. The patch may include a medicament, anesthetic, or a thermal compound. Adhesion between the patch and the flange is less than adhesion between the patch and tissue, so that the patch adheres to the shield with sufficient strength to enable the shield to slide over the needle upon withdrawal, but with insufficient strength to cause the patch to pull away from a patient's skin.

## Description

### BACKGROUND

### Technical Field

The present disclosure generally relates to a skin preparation patch for use with hypodermic syringes and, in particular, to a hydrogel preparation pad comprising one or more active agents.

### Background

Syringes and skin preparation patches, or "prep pads", are well known in the medical art. A syringe is a simple piston pump which includes a plunger that fits tightly within a cylindrical tube or barrel that is adapted to draw in and/or expel a liquid or gas through an orifice at the distal end of the barrel. A distal end of the syringe may be fitted with a hypodermic needle, a nozzle, or tubing to help direct the flow of fluid into and out of the barrel. Medical syringes are used to administer injections, withdraw fluid samples from a patient, and to insert intravenous drugs into the bloodstream. Typical usage protocol dictates that a patient's skin is cleansed and/or disinfected by swabbing a prep pad at the insertion site immediately prior to insertion of a hypodermic needle. A prep pad may include an absorbent substrate that is pre-moistened with a topical agent, such as isopropyl alcohol. Subsequent to withdrawal of a hypodermic needle, an absorbent gauze compress may be applied to the insertion site to absorb blood or other fluid exudates, and/or a bandage may be applied to protect the puncture wound.

Procedures during which a needle punctures the skin may be associated with patient discomfort and fear. Such procedures may place clinicians at risk of injury and infection due to injury arising from accidental punctures. Insertion sites and related puncture wounds may be a breeding ground for bacteria, which can cause infection and complications. Accordingly, a continuing need exists in the medical art for a syringe and prep pad that enhances patient comfort, improves procedural outcomes, and reduces the risk of injury to a clinician.

### SUMMARY

The present disclosure is directed to an injection site patch formed from a hydrogel.

One embodiment is directed to an injection site preparation patch comprises a substrate, a hydrogel on a first surface of the substrate and an adhesive on the second surface of the substrate. The hydrogel comprises an active agent comprising at least one of an antimicrobial, an antiseptic, an antibiotic, an anesthetic, a warming agent, and a cooling agent, disposed on at least a portion of the first surface.

Another embodiment is directed to a safety syringe system comprising a safety syringe and a site preparation patch. The syringe comprises a syringe body having an internal fluid reservoir, a fluid outlet disposed at the distal end of the reservoir, and a syringe annular stop disposed at a distal end of an outer surface of the syringe body. The syringe also comprises a plunger at least partially disposed within the syringe body, the plunger including a plunger rod and a distal plunger head, the plunger adapted for longitudinal movement between a proximal retracted position and a distal advanced position. The syringe also has a safety shield disposed about at least a portion of the syringe body and slidable along a longitudinal axis of the syringe body, the safety shield including a flange disposed at a distal end of the safety shield, a shield proximal stop disposed at a proximal end of an inner surface of the safety shield, and a shield distal stop disposed at a distal end of an inner surface of the safety shield. The preparation patch comprises a first side comprising a hydrogel including an active agent comprising at least one of an antimicrobial, an antiseptic, an antibiotic, an anesthetic, a warming agent, and a cooling agent, disposed on at least a portion of the first surface and adapted to adhere to a surface of a subject. The preparation patch also includes a second side comprising an adhesive, wherein the adhesion between the adhesive and the flange is less than the adhesion between the hydrogel and the surface of the subject.

Another aspect of the invention is directed to a method for operating a safety syringe system. The method comprises applying a first side of an injection site preparation patch to a surface of the subject and bringing a distal surface of a safety shield of a safety syringe into contact with a second side of the injection site preparation patch. The method further comprises advancing at least a portion of the safety syringe distally to cause a needle thereof to penetrate the surface of the subject and withdrawing the needle from the penetrated surface of the subject, wherein the safety shield remains adhered to the second adhesive side of the injection site preparation patch until a distal end of the safety shield is positioned distally of the distal end of the needle.

### BRIEF DESCRIPTION OF THE DRAWINGS

The accompanying drawings, which are incorporated in and constitute a part of this specification, illustrate embodiments of the disclosure and, together with a general description of the disclosure given above, and the detailed description of the embodiment(s) given below, serve to explain the principles of the disclosure, wherein:
Fig. 1 is a side view of an embodiment of a safety syringe illustrating a syringe body and a safety shield extending distally from the syringe body in accordance with the present disclosure;
Fig. 2 is a side view of the safety syringe shown in Fig. 1 illustrating the syringe body and the safety shield retracted to expose a needle conduit extending distally from the syringe body in accordance with the present disclosure;
Fig. 3 is a side view of the safety syringe shown in Fig. 1 illustrating details of the syringe unit with the safety shield removed;
Fig. 4 is a side view of the dispensing element of the safety syringe shown in Fig. 1;
Fig. 5 is an exploded view of the safety syringe shown in Fig. 1 illustrating the syringe body, plunger, dispensing element, and safety shield;
Fig. 6 is a perspective view of a preparation patch in accordance with the an embodiment of the present disclosure;
Fig. 7A is an edge view of the preparation patch shown in Fig. 6;
Fig. 7B is an edge view of an alternate embodiment of the preparation patch shown in Fig. 6;
Fig. 7C is an edge view of an alternate embodiment of the preparation patch shown in Fig. 6;
Fig. 8A is a side, cutaway view of a syringe system including the safety syringe shown in Fig. 1 with a hydrogel prep patch applied to a patient's skin prior to an injection being administered;
Fig. 8B is a side, cutaway view of the syringe system of Fig. 8A showing the safety shield contacting the hydrogel prep patch in accordance with the present disclosure;
Fig. 8C is a side, cutaway view of the syringe system of Fig. 8A showing a needle of the safety syringe inserted into tissue through the hydrogel patch;
Fig. 8D is a side, cutaway view of the syringe system of Fig. 8A showing a plunger moved distally;
Fig. 8E is a side, cutaway view of the syringe system of Fig. 8A showing the needle being withdrawn from tissue; and
Fig. 8F is a side, cutaway view of the syringe system of Fig. 8A showing the safety shield of the safety syringe separated from the hydrogel prep and the safety shield positioned distally of the penetrating end of the needle.

### DETAILED DESCRIPTION

Embodiments of the presently disclosed antimicrobial syringe system will now be described in detail with reference to the drawings wherein like reference numerals designate identical or corresponding elements in each of the several views. In this description, the term "proximal" is generally used to indicate the relative nearness of a referenced item to a clinician (e.g., away from the patient and/or tissue) using the assembly and the term "distal" is used to indicate the remoteness of a referenced item to a clinician using the device (e.g., toward the patient and/or tissue). As used herein, terms referencing orientation, e.g., "top", "bottom", "up", "down", "left", "right" and the like are used for illustrative purposes with reference to the figures. It is to be understood that embodiments in accordance with the present disclosure may be utilized in any orientation without limitation.

The present disclosure is directed to a flexible patch formed from a hydrogel that comprises one or more physiologically active agents, such as medicaments and/or antimicrobials. One or more active agents may be incorporated into the hydrogel and/or coated on the hydrogel surface which will contact the surface of a subject. One example of an antimicrobial is polyhexamethylene biguanide (PHMB). One example of a medicament includes an anesthetic, such as without limitation, lidocaine, wherein the patch is configured to deliver the anesthetic to the insertion site, which, in turn, may numb the insertion site thereby enhancing patient comfort. Additionally or alternatively, a hydrogel patch in accordance with the present disclosure may include a thermal modification component that is configured to warm and/or cool the insertion site. It is believed the warming and/or cooling effect of the patch on a patient's skin may promote sensory redirection, which may reduce or mask a patient's sensation of the needle insertion. The warming and/or cooling effect of the patch may also increase blood circulation in the local area and may reduce swelling associated with injections.

The preparation patch may be configured to adhere to the skin and/or to conform to the injection site. The inherent adhesive property of the hydrogel may prevent the patch from moving away from the skin. In one embodiment, the preparation patch may comprise a hydrogel disposed between two release liners. The hydrogel may have sufficient strength to adhere to a surface of a subject as well as to adhere to a surface of a safety syringe to activate the safety shield. In one embodiment, the adhesive strength between the hydrogel and the surface of the subject is greater than the adhesive strength between the hydrogel and the safety shield, thereby activating the shield and removing the shield from the hydrogel without removing the hydrogel from the surface of the subject. The portion of the safety shield may, but need not, comprise a release agent to control the adhesive force between the shield and the hydrogel. Conventional release agents may be incorporated into the shield and/or coated on a portion of the shield which contacts the hydrogel. After the safety shield is removed from the hydrogel, the preparation patch may be covered with a material, such as gauze, to create a bandage having anitmicorbial properties.

In one embodiment, the preparation patch may include a first adhesive, such as a hydrogel, on one surface of the patch to adhere to a surface of a patient and a second adhesive on a second surface of the patch opposite the first surface. The patch may include release liners on one or both sides that may be removed prior to application to expose an active agent and/or an adhesive. The first adhesive may have an adhesive strength between the first adhesive and the surface of a subject great than an adhesive strength between the second adhesive and a portion of a safety shield of a syringe so that the patch may remain with the subject upon withdrawal of the syringe. In yet another embodiment, the preparation patch may comprise a flexible substrate disposed between a first adhesive, such as a hydrogel, and a second adhesive. After the procedure (e.g., after needle withdrawal from the patient), the patch can be covered with gauze or other material to form an adhesive bandage having antimicrobial properties.

In some embodiments, a suitable hydrogel of the present disclosure may include a copolymer. Non-limiting examples of suitable copolymers may include a first monomer, such as a mixture of acrylic acid and a salt thereof, and a second monomer, such as one or more monomers of the general formula CH₂CHC(O)XR, in which X is O or NH, and R is an unsubstituted or substituted alkyl group of from about 1 to about 5 carbon atoms. The hydrogel may also include water; an electrolyte or mixture of electrolytes; a polymerization initiator; a neutralizer a such as sodium hydroxide; a penetration enhancer such as dimethylsulfoxide; optionally a humectant; optionally, a crosslinking agent; and optionally, a thickener.

An example of a suitable polymer which may be utilized in the hydrogel includes RG-63B, commercially available from Covidien. Other suitable hydrogels include those disclosed in U.S. Patent Application Publication Nos. 2009/0270709; 2009/0270710; 2011/0230816; and 2012/0041296, the entire disclosures of each of which are incorporated by reference herein for all purposes. In embodiments, the above polymers and/or hydrogels may be modified in accordance with the present disclosure, rendering them suitable for use as active agent delivery devices.

In one embodiment, the hydrogel comprises an analgesic selected from the group consisting of methyl salicylate, salicylic acid, acetaminophen, oxycodone, hydrocodone, COX-2 inhibitors, non-steroidal anti-inflammatory drugs, and combinations thereof. In another embodiment, the hydrogel comprises an anesthetic selected from the group consisting of benzocaine, bupivacaine, butesin picrate, chloroprocaine, ethyl chloride, fluori-methane, lidocaine HCl, mepivacaine, pramoxine HCl, and combinations thereof. In yet another embodiment, the hydrogel comprises a warming component selected from the group consisting of capsaicin, nonivamide, cinnamaldehyde, and combinations thereof. In yet another embodiment, the hydrogel comprises a cooling component selected from the group consisting of menthol, camphor, eucalyptol, icilin, methyl lactate, N-ethyl-p-menthane-3-carboxamide, and combinations thereof. In some embodiments, hydrogel may include an antimicrobial such as polyhexamethylene biguanide (PHMB), benzalkonium chloride and combinations thereof, and/or an anesthetic such as lidocaine, prilocaine, and combinations thereof.

During use, the preparation patch may cooperate with one or more feature of a safety syringe to passively activate (e.g., without clinician intervention) a safety needle shield. A portion of the safety shield may contact a second side of the preparation patch facing the safety shield and temporarily adhere to the patch. As a syringe needle cannula is withdrawn, at least a portion of a distal end of the shield may remain in contact with the preparation patch until the shield is fully extended over the tip of the needle cannula. Once the safety shield is fully deployed, continued withdrawal of the needle away from the surface of the subject will separate the distal portion of the shield from the second side of the preparation patch, while the hydrogel remains with the surface of the subject.

Any safety syringe having a safety feature which may be activated by contact with the preparation patch may be used. In one embodiment, the safety shield may incorporate a cylindrical member slidably disposed at a distal end of a syringe. A distal surface of the shield may include a flange adapted to temporarily adhere to the second surface of the preparation patch. In one embodiment, prior to use, the safety shield may be extended to a first, distal position wherein a distal end of the shield extends beyond a distal end of the needle cannula. The shield may be retained in the distal position frictionally. During a needle insertion procedure, the preparation patch is affixed to a patient's skin at the insertion site. To perform an injection, the flanged portion of the safety shield is brought into contact with the second side of the preparation patch. The needle is advanced through the patch, through the patient's skin, and into the patient's body. As the needle is inserted, the flange contacts the hydrogel or adhesive on second surface of the preparation patch, which, in turn, overcomes the frictional resistance between the shield and the syringe thereby causing the shield to slide proximally with respect to the syringe. Additionally, when the flange is brought into contact with the preparation patch, the second adhesive on the surface of the preparation patch causes the flange to adhere to the patch. As the needle is withdrawn by, e.g., pulling the syringe away from the insertion site, the patch continues to adhere to the flange, which, in turn, overcomes the frictional resistance between the shield and the syringe thereby causing the shield to slide distally with respect to the syringe. In this manner, the safety shield is positioned in a distal position when the needle is fully withdrawn from the body, thus protecting the exposed needle.

In another embodiment, the safety shield is not deployed prior to use so that the needle cannula is exposed prior to insertion. Upon insertion of the needle cannula into the patient, the safety shield contacts the patch at which time the hydrogel or adhesive at the second surface of the patch engages a component of the safety shield, causing the safety shield to move distally with respect to the syringe.

In some embodiments, the syringe may comprise a biasing member, such as a spring, configured to bias the safety shield distally with respect to the syringe. During insertion, the force of the biasing member is overcome to permit the shield to move proximally with respect to the syringe and to enable the needle to penetrate the insertion site. Upon withdrawal, the biasing force returns the shield to its distal position to surround the needle.

The preparation patch and/or safety syringe in accordance with the present disclosure may be utilized on any suitable injection site of a patient, during any desired procedure, including without limitation an intramuscular injection, a gastrointestinal port, a spinal catheter, stoma management, and the like. When used alone, or in combination with a passive needle safety shield, the disclosed preparation patch may provide improved comfort and care to the patient before, during, and after needle use. In one embodiment, the patch may remain with the patient for an extended period of time after injection and thereby may reduce a patient's discomfort and/or reduce the likelihood of infection after a needle insertion. In another embodiment, the patch may be removed from the patient shortly after injection.

The preparation patch may comprise flexible properties which may enable it to conform to the targeted area of the patient's skin. This, in addition to its adhesive properties, may increase contact with the patient to promote the efficacy of active agents included within the patch, e.g., lidocaine, PHMB, and the like, and enable such medicaments to continue working at the puncture site after application. One advantage of the preparation patch is that it may replace one or more prep pads commonly used during injection, such as alcohol prep pads and bandages. In addition, a syringe safety system in accordance with the present disclosure may enhance clinician safety because of the passive safety system that covers the sharp immediately or concurrently upon needle withdrawal. In contrast, prior art safety needle and syringe systems require activation by the clinician.

In one embodiment, a safety syringe includes a syringe body having an internal fluid reservoir, a fluid outlet disposed at the distal end of the reservoir, and a syringe annular stop disposed at a distal end of an outer surface of the syringe body. The syringe includes a plunger at least partially disposed within the syringe body, the plunger including a plunger rod and a distal plunger head, the plunger adapted for longitudinal movement between a proximal retracted position and a distal advanced position. A safety shield, which may be generally cylindrical in shape, is disposed about at least a portion of the syringe body and is slidable along a longitudinal axis of the syringe body. The safety shield may include a flange disposed at a distal end of the safety shield, a shield proximal stop disposed at a proximal end of an inner surface of the safety shield, and a shield distal stop disposed at a distal end of an inner surface of the safety shield.

The syringe body may include a fitting at a distal end thereof configured to operably couple in fluid communication the fluid outlet with a central lumen of a needle cannula, e.g., a hypodermic needle. In some embodiments, the syringe body includes a needle cannula having a central lumen defined therethrough that is in fluid communication with the fluid outlet. Embodiments are contemplated that employ a fixed (e.g., non-removable) needle that is attached or integral to the syringe body, as well as embodiments that are configured to accept a removable or replaceable needle that couples to the syringe assembly using a luer fitting or other suitable coupler. In some embodiments, the safety shield is slidable between a distal position configured to shield a needle disposed at a distal end of the syringe body, and a proximal position configured to expose a needle disposed at a distal end of the syringe body.

In some embodiments, a syringe annular stop may be positioned between the shield proximal stop and the shield distal stop and is adapted to limit the longitudinal excursion of the safety shield. In some embodiments, the syringe annular stop, the shield distal stop, and the shield proximal stop are arranged to limit the longitudinal excursion of the safety shield. In yet other embodiments, the syringe annular stop, the shield distal stop, and the shield proximal stop are arranged to maintain the safety shield in concentric alignment with the syringe body. In some embodiments, the syringe annular stop, the shield distal stop, and the shield proximal stop are dimensioned to impart friction between the safety shield and the syringe body such that the position of the safety shield with respect to the syringe body is maintained unless overcome by an external force, e.g., the force encountered when inserting or withdrawing the needle into, or out of, patient tissue.

One aspect of the invention is directed to a safety syringe system comprising a safety syringe and an injection site preparation patch adapted for use with the safety syringe. In some embodiments, the disclosed safety syringe includes a syringe body having an internal fluid reservoir, a fluid outlet disposed at the distal end of the reservoir, and a syringe annular stop disposed at a distal end of an outer surface of the syringe body. The safety syringe includes a plunger at least partially disposed within the syringe body, the plunger including a plunger rod and a distal plunger head, the plunger adapted for longitudinal movement between a proximal retracted position and a distal advanced position. The safety syringe includes a safety shield, which may by generally cylindrical in shape, that is disposed about at least a portion of the syringe body and slidable along a longitudinal axis of the syringe body, the safety shield including a flange disposed at a distal end of the safety shield, a shield proximal stop disposed at a proximal end of an inner surface of the safety shield, and a shield distal stop disposed at a distal end of an inner surface of the safety shield.

The disclosed safety syringe system includes an injection site preparation patch having a first side facing the surface of a subject and a second side facing a syringe. The injection site preparation patch may comprise: a hydrogel disposed between two release liners. In other embodiments, the preparation patch comprises a first surface comprising a first adhesive, such as a hydrogel, and a second surface comprising a second adhesive. The preparation patch may, but need not, comprise a flexible substrate. The adhesion between the second side of the preparation patch and a portion of the safety shield is less than the adhesion between first side of the preparation patch and the surface of the subject. By this arrangement, the injection site preparation patch will adhere to the safety shield with sufficient force to enable the safety shield to slide with respect to the syringe body upon withdrawal (e.g., slide distally to surround the needle), but not enough to cause the injection site preparation patch to pull away from the surface of a subject, such as a patient's skin.

The preparation patch of the safety syringe system may be formed of a hydrogel adapted to deliver an active agent to the surface of a subject. Active ingredients include, but are not limited to antimicrobials, antiseptics, antibiotics, anesthetics, warming agents, cooling agents and combinations thereof. In some embodiments, the active agent is selected from the group consisting of polyhexamethylene biguanide (PHMB), benzalkonium chloride (e.g., alkyldimethylbenzylammonium chloride or ADBAC), lidocaine, and prilocaine.

In some embodiments, the active agent in the hygrogel is available for delivery to the subject upon removal of the first release liner. In other embodiments, the medicament may be configured to delivery to the patient upon application of pressure to at least a portion of the injection site preparation patch.

The safety syringe system may be provided as individual components or in a kit. During use, a clinician my remove the first release liner and apply the hydrogel patch to a surface of a subject at the intended insertion site. The clinician may then remove the second release liner, if present, and contact at least a portion of the safety shield of the syringe with the patch, thereby inserting the needle of the syringe into the patient. After injecting the patient with the desired medicament from the syringe, the clinician may withdraw the needle from the patient, while at least a portion of the safety shield remains in contact with the hydrogel. The clinician continues to withdraw the needle from the patient thereby fully extending the safety shield over the tip of the needle. The clinician then removes then moves the syringe and safety shield away from the patient for proper disposal of the sharps. If it is desired that the patch remain in place for a predetermined period of time, the clinician may cover the patch with gauze or other bandage, in which case, the patient will remove the patch when desired. Alternatively, the patch may be removed by the clinician immediately after the injection and withdrawal of the needle from the patient.

In yet another embodiment, the site preparation patch may be removably attached to the safety shield prior to use. The clinician may then remove a release liner from the first surface of the patch, thereby exposing the hydrogel. The clinician may then place the preparation patch in contact with the subject, while in the same motion bringing the needle guard and needle into place, thereby reducing the number of steps prior to injection. The clinician may then inject or withdraw the fluid from the syringe and remove the syringe causing the needle guard to advance over the tip of the needle prior to being removed from the second surface of the site preparation patch. In yet another embodiment, the site patch which is removable attached to the safety shield of the syringe may also include a sterile barrier, so the safety shield and preparation patch form a sterile compartment about the needle cannula.

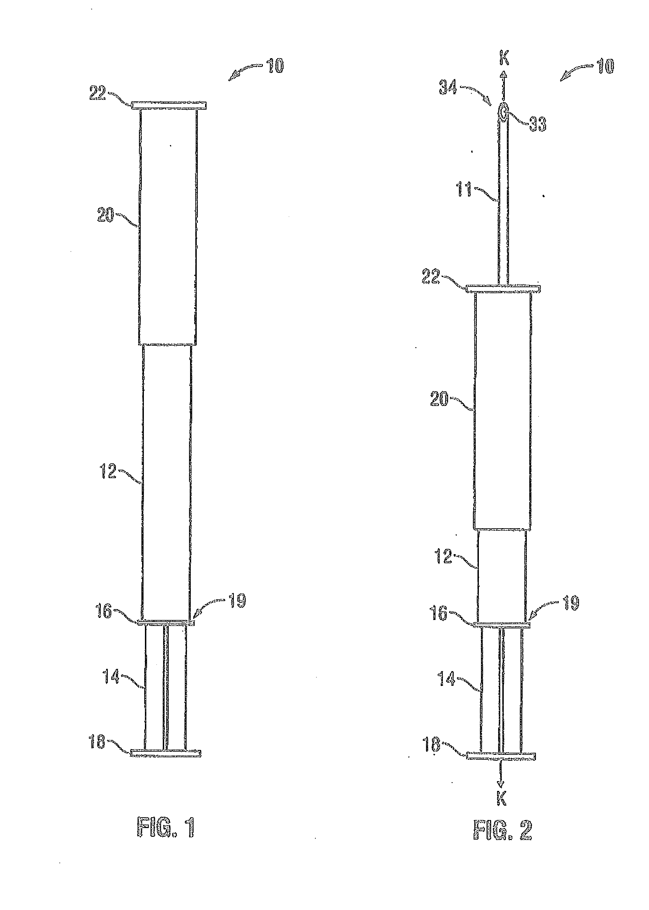

Referring now to Figs. 1, 2 and 3, a safety syringe 10 in accordance with at least one aspect of the present disclosure is shown. Safety syringe 10 is configured such that the sharp, tissue-penetrating needle 11 is shielded by a safety shield 20 from inadvertent contact with an unintended surface. Syringe assembly 10 may be utilized to deliver medicinals, including antibiotics, pain medication, therapeutic drugs, and the like. Alternatively, syringe assembly 10 may be utilized to withdraw fluids from a subject. In other embodiments, syringe assembly 10 may be used to deliver a flushing solution, e.g., saline or the like through an access device, such as an intravenous access port.

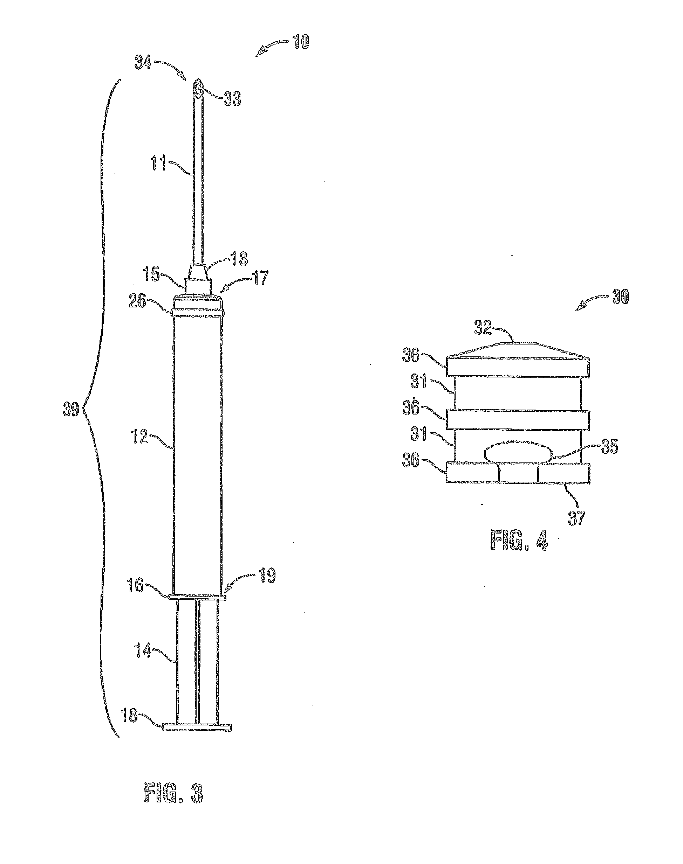

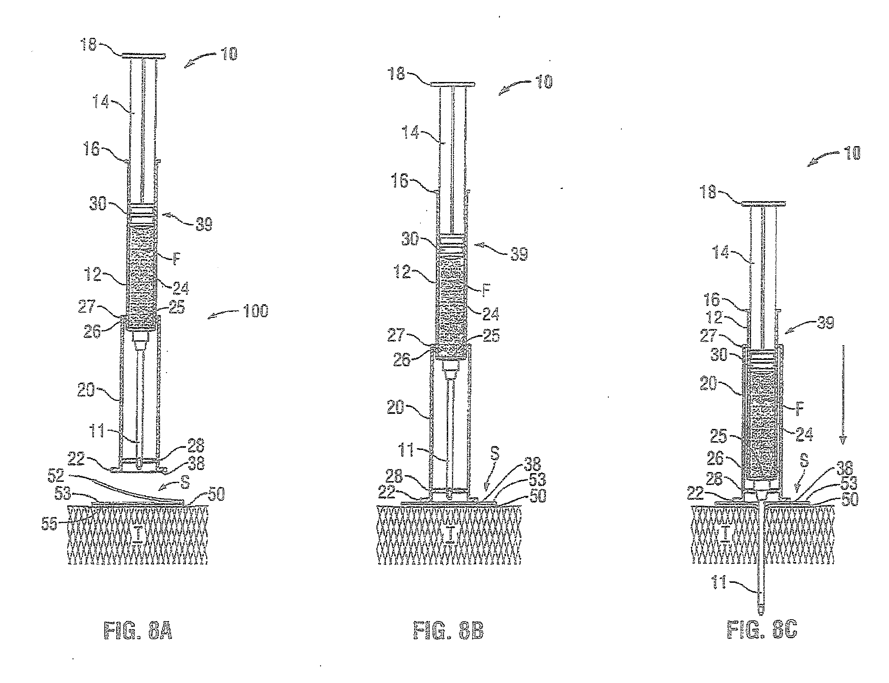

Safety syringe 10 includes a syringe body 12, a plunger 14 at least partially received within syringe body 12, a needle 11 which is mounted to syringe body 12, and a safety shield 20 slidably positioned about at least a distal end 17 of syringe body 12. Distal end 17 of syringe body 12 includes a needle hub 15, which may, in one embodiment, include a luer connector or luer lock configured to operably engage a corresponding needle fitting 13 that is fixed to needle 11. Syringe body 12 defines fluid chamber or internal reservoir 24 (see Fig. 8A et seq.), which is adapted to contain a fluid "F". Disposed at a proximal end of syringe body 12 is flange 16, which may be integrally formed with syringe body 12. Flange 16 may be relatively enlarged to facilitate engagement by the clinician. Distal end 17 further defines fluid outlet 25 that is in fluid communication with internal reservoir 24 and an internal portion of needle 11. As depicted in Fig. 2, syringe assembly 10 defines a longitudinal axis "K" extending along length of syringe body 12. Fig. 3 shows syringe unit 39, e.g., the syringe assembly 10 with safety shield 20 removed for clarity. Syringe unit 39 includes needle 11, syringe body 12, and plunger 14. Syringe unit 39 is configured to slide with respect to safety shield 20, that is, safety shield 20 is dimensioned to slidably receive at least a distal portion of syringe unit 39, i.e., a distal portion of syringe unit syringe body 12.

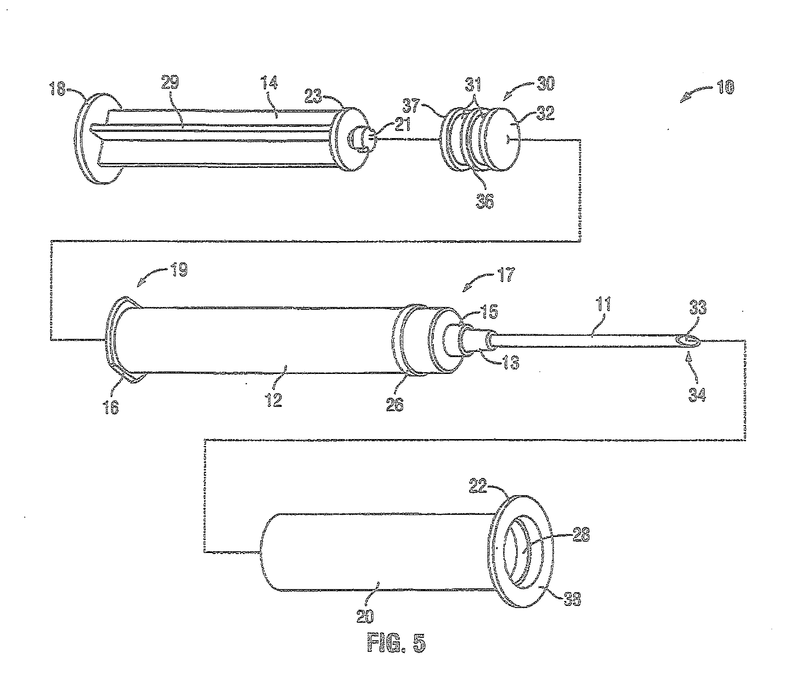

With attention now to Figs. 3 and 5, plunger 14 includes proximal plunger flange 18, an elongated plunger rod 29 extending distally from plunger flange 18, a plunger head 23 at a distal end of plunger rod 29, and dispensing element 30 that is fixed to plunger head 23. Plunger flange 18 may be enlarged to facilitate engagement by a clinician. Plunger rod 29 may have any cross-sectional configuration. In the embodiments depicted in Figs. 1-3 and Fig. 5, plunger rod 29 has a cruciform cross-section. Plunger head 23 may be disc-shaped having a cross-section or outer diameter which generally corresponds to an internal diameter of syringe body 12. Plunger head 23 may further include a mount 21 extending distally therefrom and configured to be fixedly received within a corresponding receptacle 35 defined within a proximal end 37 of dispensing element 30 to secure dispensing element 30 to plunger head 21. Dispensing element 30 may be formed from elastomeric material or the like. Dispensing element 30 includes, in some embodiments, a circular cross-sectional dimension, which generally approximates an inner diameter of syringe body 12. Dispensing element 30 is adapted to traverse, e.g., longitudinally move, with plunger 14 within syringe reservoir 18 as plunger 14 is moved to withdraw or dispense fluids into or out of fluid outlet 25 of syringe body 12 and/or needle 11.

With reference to Figs. 4 and 5, dispensing element 30 may include annular recesses 31 to reduce friction between dispensing element 30 and the internal wall of syringe body 12 to facilitate movement of dispensing element 30 through syringe body 12. Annular recesses 31 are defined between adjacent ribs 36 of dispensing element 30. Dispensing element 30 may include a distal conical face 32, which contacts fluid "F" within reservoir 34. Advantageously, the elastomeric and/or ribbed construction of dispensing element 30 promotes a fluid-tight seal between the movable proximal end of reservoir 25 and the internal wall of syringe body 12 and the outer surface of dispensing element 30.

Referring again to Figs. 2-3 and Fig. 5, needle cannula 11 includes central lumen 33 which extends the length of the needle 11 and is in fluid communication with fluid outlet 25 of syringe body 12. Needle cannula 11 includes penetrating end 34 which is adapted to pierce tissue, e.g., for entry within a vessel. Needle 11 further includes fitting 13 adjacent its proximal end. Fitting 13 couples with connector 15 of syringe body 12 to operably couple needle 11 to the syringe body 12. Connector 15 may establish a releasable connection with needle 11 and/or fitting 13 through a luer lock mechanism or the like, or may establish a more permanent connection between needle 11 and syringe body 12 with adhesives, cements, welding, etc. Alternatively, needle 11 may be secured directly to or within the needle hub 15 using adhesives or the like, thus obviating the need for a fitting 13.

As shown in Fig. 3, Fig. 5, and Figs 8A et seq., syringe body 12 includes a syringe annular stop 26 that cooperates with a shield proximal stop 27 and a shield distal stop 28 disposed on safety shield 20 to limit the extent of longitudinal movement (e.g., distally and proximally) of safety shield 20 with respect to syringe body 12. In the present embodiment, a shield annular stop 28 disposed at a generally distal end of safety shield 20 cooperates with syringe annular stop 26 to limit the proximal extent of movement of safety shield 20 with respect to syringe body 12. A shield proximal stop 27 disposed at a generally proximal end of safety shield 20 cooperates with syringe annular stop 26 to limit the distal extent of movement of safety shield 20 with respect to syringe body 12.

In some embodiments, syringe annular stop 26, shield distal stop 28, and shield proximal stop 27 may be configured to maintain concentric alignment between safety shield 20 and syringe body 12. Additionally or alternatively, annular stop 26, shield annular stop 28, and shield proximal stop 27 may be dimensioned to impart sufficient friction between safety shield 20 and syringe body 12 such that the position of safety shield 20 with respect to syringe body 12 is maintained unless overcome by a suitable external force or action, e.g., performing an injection. Safety shield 20 includes at a distal end thereof a shield flange 22 that is adapted to contact tissue (e.g., the skin of a patient) and/or a prep patch adhesively attached to tissue as described hereinbelow.

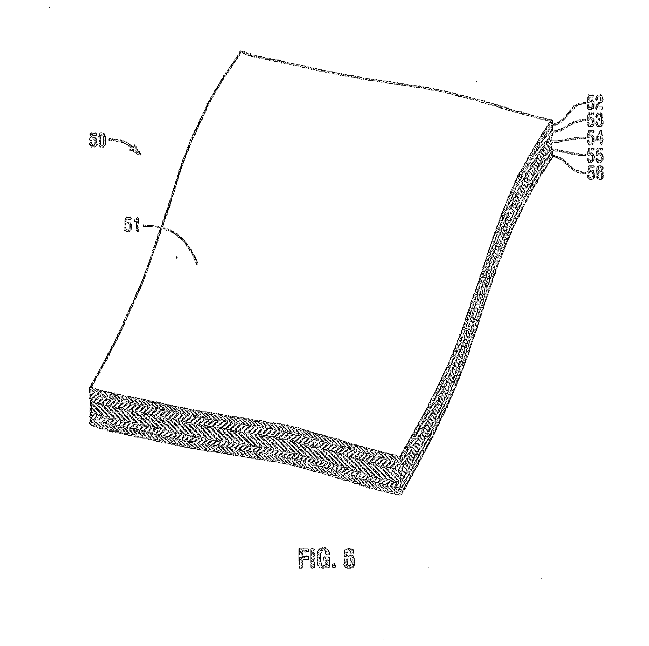

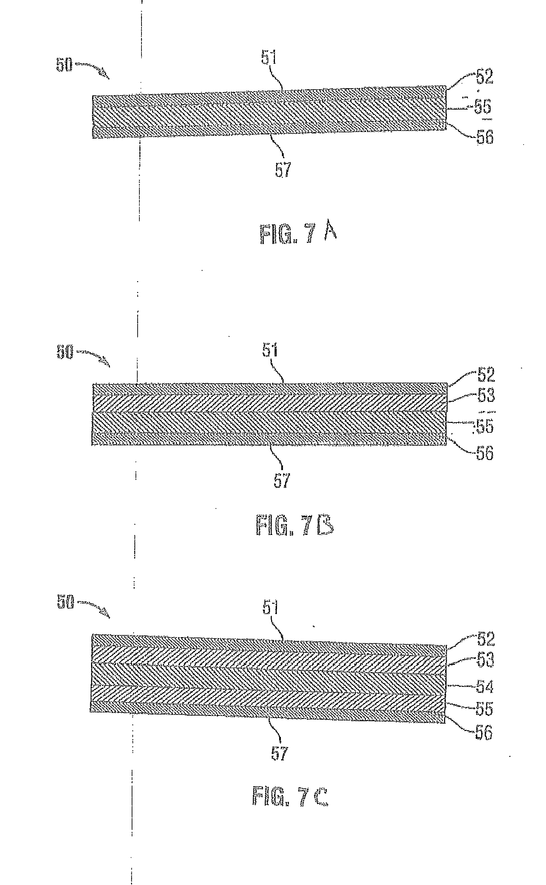

Turning now to Figs. 6 through 7C, the disclosed safety syringe system includes preparation patch 50 that is adapted to be applied to an injection site. The patch 50 includes a tissue-facing first side 57 and a syringe-facing second side 51. As seen in Fig. 7A, patch 50 comprises a hydrogel 55 disposed between two release liners 52, 56. In another embodiment shown in Fig. 7B, patch 50 comprises a first side comprising a hydrogel and a second side comprising an adhesive. A first release liner 56 is adjacent the hydrogel and a second release liner 52 is adjacent the adhesive. In yet another embodiment shown in Fig. 7C, the patch 50 site preparation patch 50 comprises a substrate 54; a hydrogel 55 adjacent a first side of the substrate 54 and a syringe adhesive 53 adjacent a second side of the substrate. A removable syringe adhesive release liner 52 is disposed upon syringe adhesive layer 53, and a removable hydrogel release liner 56 is disposed upon hydrogel layer 55. Flexible substrate 54 may impart sufficient flexibility to the patch 50 to enable the patch 50 to conform to the contours and irregularities typically seen at an injection site. The flexible and/or resilient nature of the substrate 54 enables patch 50 to be adhesively attached to and move with a patient's skin without compromising the adhesive bond between patch 50 and a patient's skin. In another aspect, the elastomeric properties of the substrate 54 may support easy penetration by needle cannula 11 and reduce needle cannula 11 dulling. Additionally or alternatively, the cohesive properties of the substrate 54 and/or the hydrogel may promote self-closure of the needle puncture that remains after a needle is withdrawn from the patch, which further protects the injection site from contaminants, infection, and the like.

Substrate 54 may be formed of any flexible material, such as cloth, scrim, foam, and combinations thereof. The substrate may, but need not, comprise an antimicrobial and/or a medicatment, including but not limited to, an antiseptic, an antibiotic, an anesthetic, a warming agent, a cooling agent and combinations thereof. The hydrogel 55 may include an antimicrobial and/or a medicament, including without limitation one or more of an antiseptic, an antibiotic, an anesthetic, a warming agent, a cooling agent. In some embodiments, substrate 54 and/or hydrogel 55 may include polyhexamethylene biguanide, benzalkonium chloride, lidocaine, and/or prilocaine. In one embodiment, the substrate and/or hydrogel layer 55 comprises an analgesic selected from the group consisting of methyl salicylate, salicylic acid, acetaminophen, oxycodone, hydrocodone, COX-2 inhibitors, non-steroidal anti-inflammatory drugs, and combinations thereof. In another embodiment, the substrate 54 and/or hydrogel layer 55 comprises an anesthetic selected from the group consisting of benzocaine, bupivacaine, butesin picrate, chloroprocaine, ethyl chloride, fluori-methane, lidocaine HCl, mepivacaine, pramoxine HCl, and combinations thereof. In yet another embodiment, the substrate and/or the hydrogel 55 comprises a warming component selected from the group consisting of capsaicin, nonivamide, cinnamaldehyde, and combinations thereof. In yet another embodiment, the substrate and/or the hydrogel 55 comprises a cooling component selected from the group consisting of menthol, camphor, eucalyptol, icilin, methyl lactate, N-ethyl-p-menthane-3-carboxamide, and combinations thereof.

In some embodiments, the active agent is configured to release and/or activate upon application of patch 50 to tissue, e.g., skin. Alternatively, the active agent is configured to release and/or activate upon removal of tissue release liner 56 from patch 50 (e.g., by peeling hydrogel release liner 56 from hydrogel layer 55). In some embodiments, the active agent is configured to release and/or activate upon application of pressure to patch 50 (e.g., by pressing onto skin).

Referring to Figs. 8A-8F, cross-sectional views of syringe assembly 10 and preparation patch 50 are presented that illustrate an embodiment of a method of use of the disclosed safety syringe system 100, and respective movements of safety shield 20 and plunger 14. The embodiments of the present disclosure are constructed such that safety shield 20 may be extended distally to a first position (e.g., a shielding position) as illustrated in Fig. 8A, thereby protecting needle 11 from inadvertent contact with unintended surface(s).

Continuing with reference to Fig. 8A, prior to use, hydrogel release liner 56 is removed from preparation patch 50 to expose hydrogle 55, which, in turn enables the application of preparation patch 50 to tissue "T" at an injection site "S". Generally, a clinician may apply preparation patch 50 to tissue "T" using any suitable technique, e.g., pressing or rolling preparation patch 50 onto injection site "S". In embodiments where preparation patch 50 includes one or more active agents, the one or more active agents are released onto tissue "T" and may become available for absorption thought the tissue. For example, an antimicrobial active agent may be provided on preparation patch 50 to disinfect, cleanse, etc. injection site "S". In some embodiments, an anesthetic medicament may be provided on preparation patch 50 to numb injection site "S", which, in turn, may enhance patient comfort during needle insertion. In some embodiments, a heating agent or a cooling agent may be provided on preparation patch 50 to provide a sensory effect or distraction to the patient at injection site "S", which, in turn, may enhance patient comfort during needle insertion.

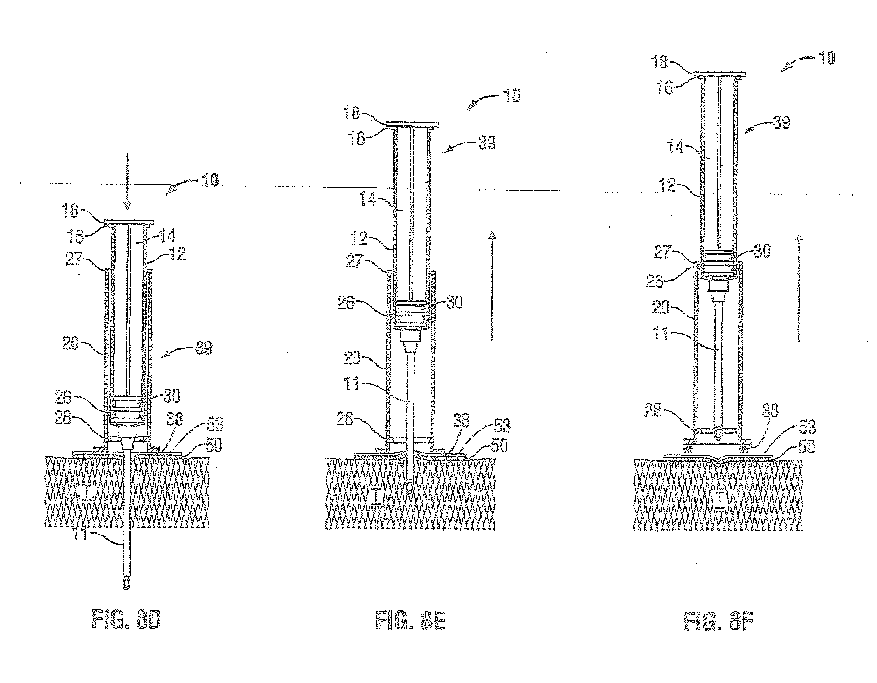

Syringe adhesive release liner 52 may be removed (e.g., peeled) from preparation patch 50 to expose the second side of patch 50, which may include adhesive 53 or hydrogel 55. The adhesive properties of the first side of patch 50 are sufficient to enable a distal surface 38 of shield flange 22 to adhere to preparation patch 50 during application and withdrawal of needle 11 from tissue "T", thereby ensuring that safety shield 20 returns to the first position as the safety syringe 10 is pulled away from the injection site "S" (Fig. 8E) after use. As safety shield 20 reaches the full distal extent thereof, e.g., as syringe annular stop 28 abuts shield proximal stop 27, the adhesive properties of the secon side of patch 50 enable the continued proximal motion of safety syringe 10 to cause distal surface 38 of shield flange 22 to release from preparation patch 50 (Fig. 8F) without compromising the adhesion of hydrogel 55 to tissue "T" and maintaining safety shield 120 in the first (e.g., shielding) position.

With reference now to Fig. 8B, safety syringe 10 is brought into contact with hydrogel patch 50 that is affixed to injection site "S". In the present embodiment, safety syringe 10 is prepared to deliver an injection, e.g., plunger 14 is in a relatively proximal position, and reservoir 24 contains a fluid "F" that is to be injected into tissue "T". In other embodiments wherein safety syringe is prepared for withdrawal (e.g., to obtain a blood sample, or other biofluid sample), plunger 14 may be in a relatively distal position. As safety syringe 10 is brought into contact with preparation patch 50, distal surface 38 of shield flange 22 contacts, and adheres to, the first side of patch 50 via either the second adhesive or the hydrogel.

In Fig. 8C, syringe unit 39 is moved distally within safety shield 20 to insert needle 11 into tissue "T". Since flange 22 of safety shield 20 is in contact with preparation patch 50, which is fixed to the patient's tissue "T", syringe unit 39 moves distally within safety shield 20, which, in turn, causes needle cannula 11 to contact, and subsequently pierce, preparation patch 50, and to penetrate into the targeted tissue "T". Syringe annular stop 26 may approach and/or contact safety shield stop 28.

As shown in Fig. 8D, plunger 14 is advanced distally within syringe body 12 by, e.g., a clinician. During advancement of plunger 14 within syringe body 12, dispensing element 36 causes the fluid "F" within internal reservoir 24 to be dispensed through fluid outlet 25 and into needle 11. Plunger 14 is continually advanced to an actuated or fully advanced position, depicted in Fig. 8D, to expel the volume of fluid "F" from internal reservoir 24 into tissue "T".

As illustrated in Figs. 8E and 8F, after fluid "F" has been dispensed, syringe unit 39 is moved proximally which causes needle cannula 11 to withdraw from tissue "T". Advantageously, the adhesion between distal surface 38 and the second side of patch 50 maintains safety shield 20 in a distal position, e.g., in contact with the tissue "T" as syringe unit 39 moves proximally. As syringe unit 39 continues to move proximally, syringe annular stop 26 approaches, and eventually engages, shield proximal stop 27, causing the proximal withdrawal force applied to syringe unit 39 by a clinician to be coupled to safety shield 20. In turn, the proximal withdrawal force overcomes the adhesion between distal surface 38 and the second side of patch 50, causing the safety shield 20 to disengage from preparation patch 50 in a fully distal position in relation to syringe unit 39, and shielding the needle cannula 11 from inadvertent contact.

Preparation patch 50 may be left in place on tissue "T", and optionally covered with gauze, bandage, or other suitable material(s) to provide continued administration of the active agents included in patch 50, such as without limitation, antimicrobials, antiseptics, anesthetics, cooling agents, and/or warming agents to tissue "T", e.g., the patient.

The described embodiments of the present disclosure are intended to be illustrative rather than restrictive, and are not intended to represent every embodiment of the present disclosure. The steps of a method disclosed herein may be performed in a different order than that described, and/or the operations performed within an individual step or steps may be desirably be combined into a single step without departing from the scope and spirit of said method. Further variations of the above-disclosed embodiments and other features and functions, or alternatives thereof, may be made or desirably combined into many other different systems or applications without departing from the spirit or scope of the disclosure as set forth in the following claims both literally and in equivalents recognized in law.

## Claims

1. An injection site preparation patch, comprising:
a substrate having a first surface and a second surface opposite the first surface;
a hydrogel comprising an active agent comprising at least one of an antimicrobial, an antiseptic, an antibiotic, an anesthetic, a warming agent, and a cooling agent, disposed on at least a portion of the first surface; and
an adhesive disposed on at least a portion of the second surface.

2. The injection site preparation patch in accordance with claim 1, wherein the adhesive strength of the adhesive is less than the adhesive strength of the hydrogel.

3. The injection site preparation patch in accordance with claim 1, wherein the substrate is formed from a flexible material.

4. The injection site preparation patch in accordance with claim 1, wherein the antimicrobial is selected from the group consisting of polyhexamethylene biguanide, benzalkonium chloride and combinations thereof.

5. The injection site preparation patch in accordance with claim 1, wherein the anestetic is selected from the group consisting of benzocaine, bupivacaine, butesin picrate, chloroprocaine, ethyl chloride, fluori-methane, lidocaine HCl, mepivacaine, pramoxine HCl, and combinations thereof.

6. The injection site preparation patch in accordance with claim 1, wherein the analgesic is selected from the group consisting of methyl salicylate, salicylic acid, acetaminophen, oxycodone, hydrocodone, COX-2 inhibitors, non-steroidal anti-inflammatory drugs, and combinations thereof.

7. The injection site preparation patch in accordance with claim 1, wherein the warming agent is selected from the group consisting of capsaicin, nonivamide, cinnamaldehyde, and combinations thereof.

8. The injection site preparation patch in accordance with claim 1, wherein the cooling agent is selected from the group consisting of menthol, camphor, eucalyptol, icilin, methyl lactate, N-ethyl-p-menthane-3-carboxamide, and combinations thereof.

9. The injection site preparation patch in accordance with claim 1, further comprising a release liner disposed on the hydrogel, wherein active agent is configured to activate upon removal of the release liner from the hydrogel.

10. The injection site preparation patch in accordance with claim 4, wherein the active agent is configured to activate upon application of pressure to at least a portion of the injection site preparation patch.

11. A safety syringe system, comprising:
a safety syringe, comprising:
a syringe body having an internal fluid reservoir, a fluid outlet disposed at the distal end of the reservoir, and a syringe annular stop disposed at a distal end of an outer surface of the syringe body;
a plunger at least partially disposed within the syringe body, the plunger including a plunger rod and a distal plunger head, the plunger adapted for longitudinal movement between a proximal retracted position and a distal advanced position; and
a safety shield disposed about at least a portion of the syringe body and slidable along a longitudinal axis of the syringe body, the safety shield including a flange disposed at a distal end of the safety shield, a shield proximal stop disposed at a proximal end of an inner surface of the safety shield, and a shield distal stop disposed at a distal end of an inner surface of the safety shield; and
an injection site preparation patch comprising:
a first side comprising a hydrogel including an active agent comprising at least one of an antimicrobial, an antiseptic, an antibiotic, an anesthetic, a warming agent, and a cooling agent, disposed on at least a portion of the first surface and adapted to adhere to a surface of a subject; and
a second side comprising an adhesive,
wherein the adhesion between the adhesive and the flange is less than the adhesion between the hydrogel and the surface of the subject.

12. The safety syringe system in accordance with claim 11, further comprising a flexible substrate disposed between the hydrogel and the adhesive.

13. The safety syringe system in accordance with claim 11, wherein the anestetic is selected from the group consisting of benzocaine, bupivacaine, butesin picrate, chloroprocaine, ethyl chloride, fluori-methane, lidocaine HCl, mepivacaine, pramoxine HCl, and combinations thereof.

14. The safety syringe system in accordance with claim 11, wherein the analgesic is selected from the group consisting of methyl salicylate, salicylic acid, acetaminophen, oxycodone, hydrocodone, COX-2 inhibitors, non-steroidal anti-inflammatory drugs, and combinations thereof.

15. The safety syringe system in accordance with claim 11, wherein the warming agent is selected from the group consisting of capsaicin, nonivamide, cinnamaldehyde, and combinations thereof.

16. The safety syringe system in accordance with claim 11, wherein the cooling agent is selected from the group consisting of menthol, camphor, eucalyptol, icilin, methyl lactate, N-ethyl-p-menthane-3-carboxamide, and combinations thereof.

17. The safety syringe system in accordance with claim 11, further comprising a release liner disposed on the hydrogel, wherein the active agent is configured to activate upon removal of the release liner from the hydrogel.

18. The safety syringe system in accordance with claim 11, wherein the active agent is configured to activate upon application of pressure to at least a portion of the injection site preparation patch.

19. A method for operating a safety syringe system, comprising:
applying a first side of an injection site preparation patch to a surface of the subject;
bringing a distal surface of a safety shield of a safety syringe into contact with a second side of the injection site preparation patch;
advancing at least a portion of the safety syringe distally to cause a needle thereof to penetrate the surface of the subject; and
withdrawing the needle from the penetrated surface of the subject, wherein the safety shield remains adhered to the second adhesive side of the injection site preparation patch until a distal end of the safety shield is positioned distally of the distal end of the needle.

20. The method for operating a safety syringe system in accordance with claim 19, further comprising:
removing a release liner from the first adhesive side of an injection site preparation patch; and
removing a release liner from the second adhesive side of an injection site preparation patch.

21. The method for operating a safety syringe system in accordance with claim 19, further comprising providing an injection site preparation patch comprising hydrogel comprising an active agent comprising at least one of an antimicrobial, an antiseptic, an antibiotic, an anesthetic, a warming agent, and a cooling agent.

22. The method for operating a safety syringe system in accordance with claim 20, wherein.

23. The method for operating a safety syringe system in accordance with claim 19, further comprising advancing a syringe plunger distally to cause a fluid contained in a syringe reservoir to be dispensed through a central lumen of the needle.

24. The method for operating a safety syringe system in accordance with claim 19, further comprising applying at least one of a gauze or a bandage to the injection site preparation patch.

25. A safety syringe, comprising:
a syringe body having an internal fluid reservoir, a fluid outlet disposed at the distal end of the reservoir, and a syringe annular stop disposed at a distal end of an outer surface of the syringe body;
a plunger at least partially disposed within the syringe body, the plunger including a plunger rod and a distal plunger head, the plunger adapted for longitudinal movement between a proximal retracted position and a distal advanced position; and
a generally cylindrical safety shield disposed about at least a portion of the syringe body and slidable along a longitudinal axis of the syringe body, the safety shield including a flange disposed at a distal end of the safety shield, a shield proximal stop disposed at a proximal end of an inner surface of the safety shield, and a shield distal stop disposed at a distal end of an inner surface of the safety shield.

26. The safety syringe in accordance with claim 25, wherein the syringe body further comprises a fitting at a distal end thereof configured to operably couple in fluid communication the fluid outlet with a central lumen of a needle.

27. The safety syringe in accordance with claim 25, wherein the syringe body further comprises a needle having a central lumen defined therethrough, the central lumen in fluid communication with the fluid outlet.

28. The safety syringe in accordance with claim 25, wherein the safety shield is slidable between a distal position configured to shield a needle disposed at a distal end of the syringe body, and a proximal position configured to expose a needle disposed at a distal end of the syringe body.

29. The safety syringe in accordance with claim 25, wherein the syringe annular stop is captured between the shield proximal stop, and the shield distal stop and adapted to limit the longitudinal excursion of the safety shield.

30. The safety syringe in accordance with claim 25, wherein the syringe annular stop, the shield distal stop, and the shield proximal stop are arranged to limit the longitudinal excursion of the safety shield.

31. The safety syringe in accordance with claim 25, wherein the syringe annular stop, the shield distal stop, and the shield proximal stop are arranged to maintain the safety shield in concentric alignment with the syringe body.

32. The safety syringe in accordance with claim 25, wherein the syringe annular stop, the shield distal stop, and the shield proximal stop dimensioned to impart friction between the safety shield and the syringe body such that the position of the safety shield with respect to the syringe body is maintained unless overcome by an external force.

33. The safety syringe in accordance with claim 25, further comprising an injection site preparation patch, the patch comprising a first side and a second side, wherein at least a portion of the second side is removable attached to a distal surface of the flange.

34. The safety syringe in accordance with claim 33, wherein the injection site preparation patch further comprises a sterility barrier.
